# FASCICULE DE BREVET EUROPEEN

(11) **EP 1 631 263 B1**
(45) Date de publication et mention de la délivrance du brevet: **26.02.2014**
(21) Numéro de dépôt: 04767267.0
(22) Date de dépôt: 04.06.2004
(51) Int. Cl.: A61K 9/24, A61K 9/20, A61K 9/00

(54) **COMPRIME ORODISPERSIBLE MULTICOUCHE**
ORAL DISPERGIERBARE MEHRSCHICHTIGE TABLETTE
ORALLY-DISPERSIBLE MULTILAYER TABLET

(30) Priorité: 06.06.2003 FR 0306900; 30.06.2003 US 610668
(43) Date de publication de la demande: 08.03.2006
(73) Titulaire: ETHYPHARM, 92213 Saint-Cloud Cédex (FR)
(72) Inventeur: OURY, Pascal, F-78150 Le Chesnay (FR); LAMOUREUX, Gael, F-28210 Le Boullay Thierry (FR); HERRY, Catherine, F-27810 Marcilly-sur-Eure (FR); PREVOST, Yann, F-28170 Tremblay-Les-Villages (FR)
(74) Mandataire: Cabinet Plasseraud
(86) Numéro de dépôt international: PCT/FR2004/001400
(87) Numéro de publication internationale: WO 2004/110411

(56) Documents cités:
- WO-A-94/06416
- WO-A-03/017985
- US-A- 5 236 713
- DATABASE WPI Section Ch, Week 200121 Derwent Publications Ltd., London, GB; Class B07, AN 2001-205140 XP002268530 & JP 2000 336027 A (LION CORP) 5 décembre 2000 (2000-12-05)
- "Pharmacopée Européenne", 2001, Direction Européenne de la Qualité du Médicament & des Soins de Santé vol. 4 * pages 562,563, * * page 628 *

## Description

La présente invention concerne un comprimé orodispersible multicouche, et son procédé de préparation.

Par comprimé orodispersible, on entend un comprimé destiné à se désintégrer ou à se solubiliser dans la bouche, sans mastication, au contact de la salive, en moins de 60 secondes, de préférence en moins de 40 secondes, en formant une suspension de particules, aisée à avaler.

Le temps de désintégration correspond à la durée entre le moment de la mise en place du comprimé sur la langue et le moment de la déglutition de la suspension résultant de la désintégration ou la dissolution du comprimé.

Ce type de comprimé est par exemple, décrit dans les documents EP 548356, EP 636364, EP 1003484, EP 1058538, WO 98/46215, WO 00/06126, WO 00/27357 et WO 00/51568.

Une fois avalées, les particules de substance active libèrent la substance active dans la partie basse du tractus gastro-intestinal.

Le comprimé orodispersible, de par sa simplicité d'utilisation, est parfaitement adapté au traitement ambulatoire, plus particulièrement pour certains patients et notamment les personnes âgées ou les jeunes enfants, qui connaissent des difficultés de déglutition telles qu'il leur est désagréable, voire impossible d'ingérer des comprimés ou des gélules, même avec une prise simultanée de liquide.

Il est estimé que 50 % de la population connaît de telles difficultés, avec comme conséquence éventuelle, la non-prise du médicament prescrit et ainsi une forte incidence sur l'efficacité du traitement (H. Seager, 1998, J. Pharm. Pharmacol. 50, 375-382).

Ces difficultés de déglutition sont bien évidemment exacerbées lorsque plusieurs médicaments doivent être pris tout au long de la journée, multipliant alors le nombre de prises.

Des comprimés orodispersibles comprenant des associations fixes de substances actives représenteraient une solution pour améliorer l'observance des traitements de longue durée, dans le cas de pathologies chroniques qui touchent notamment les patients les plus âgés ou les enfants.

Des tentatives pour réaliser de tels comprimés ont déjà été réalisées, en comprimant par exemple un mélange unique comprenant à la fois des excipients de compression et des substances actives. Cependant ces comprimés présentent certains inconvénients, notamment une hétérogénéité des teneurs en chacune des substances actives, ou un risque d'incompatibilité entre les différents composants du comprimé, substances actives ou excipients.

En effet, une première difficulté technique est d'obtenir une homogénéité des teneurs de chaque substance active, tout au long du procédé de mise en forme, en l'occurrence la compression du mélange de poudres comprenant tous les composants dudit comprimé.

Les mélanges de poudre sont généralement complexes à maîtriser car ils sont constitués de plusieurs populations de particules de substances actives et d'excipients, chacune ayant ses propres caractéristiques de taille, de densité ou de formes.

De cette hétérogénéité découle un risque accru de ségrégation, qui se traduit par un démélange progressif de certaines populations de particules, au cours du stockage ou dans la trémie d'alimentation de la machine à comprimer.

La forme unitaire finale présente alors une teneur hautement variable en chacune des substances actives, et des caractéristiques intrinsèques de dureté, de désintégration ou de palatabilité significativement différentes dans un même lot.

Le choix minutieux des populations de substances actives et d'excipients ne suffit pas à éliminer complètement ce risque.

Par ailleurs, d'autres solutions, applicables aux comprimés orodispersibles, ont été proposées pour améliorer l'unité de teneur, par exemple par la Demanderesse dans la demande de brevet FR 03 01308 (non publiée à ce jour), mais celles-ci ne sont pas complètement satisfaisantes pour limiter les risques d'incompatibilité.

En effet, une seconde difficulté technique pour réaliser des comprimés comprenant une association de principes actifs est le choix des substances actives et des excipients pouvant être mis en oeuvre ensemble, en raison d'un risque d'incompatibilité entre les substances actives elles-mêmes ou entre une substance active et des excipients, ce risque augmentant quand le nombre de composants présents dans le comprimé est plus élevé.

Afin de réduire ces risques d'incompatibilité, des solutions ont été proposées, notamment par la préparation de comprimés multicouches. De tels comprimés sont décrits depuis de très nombreuses années (Abrégé de Pharmacie Galénique, Le Hir, 3ème Ed., p. 269, Evaluation of bilayer tablet machines - A case study. S.P. Li, M.G. Karth, K.M. Feld, L.C. Di Paolo, C.M. Pendharkar, R.O. Williams, Drug Dev. Ind. Pharm., 21 (5), 571 - 590 (1995)).

A titre d'exemple, le document WO 03/017985 décrit un comprimé dispersible multicouche pour utilisation orale, ledit comprimé comprenant du clavulanate et de l'amoxicilline en deux couches fabriquées à partir de deux formulations distinctes. Le document WO 94/06416 décrit des comprimés comprenant au moins trois couches, deux desdites couches comprenant chacune la même ou une différente substance active, lesdits comprimés étant préparés par compression d'au moins trois granulés. Le document US 5,236,713 décrit une préparation à libération contrôlée pour une libération périodique d'un ingrédient actif, ladite préparation comprenant plusieurs couches. Le document JP 2000/336027 décrit une méthode pour éviter le clivage des couches d'un comprimé multicouche, tel qu'un comprimé à croquer, en ajustant le ratio du diamètre particulaire moyen des granules présents dans les deux couches du comprimé.

Cependant, aucun des documents ci-dessus cités ne décrit un comprimé multicouche orodispersible, à savoir destiné à se désintégrer ou à se solubiliser facilement dans la bouche, sous l'action de la salive.

Les comprimés multicouches sont formés d'au moins deux couches adhérant entre elles par une surface.

Les couches du comprimé possèdent chacune leur propre composition, et sont successivement formées par un cycle de compression, ce qui limite à la fois les risques d'hétérogénéité de teneur et d'incompatibilité physico-chimique.

Ce type de comprimé nécessite cependant des ajustements de formulation pour assurer la cohésion des différentes couches.

Ce but est habituellement atteint par l'application de forces de compression élevées aboutissant à des comprimés ayant des valeurs de dureté souvent bien supérieures à 100 N, ou par la présence d'un liant dans au moins une des couches du comprimé, en quantité efficace pour promouvoir l'adhésion entre les couches.

De plus, la préparation d'un comprimé multicouche nécessite de répéter l'application de forces de compression sur chaque mélange de poudre.

Ces conditions ne sont donc favorables, ni dans le cas de comprimés destinés à se désintégrer rapidement, ni dans le cas de substances actives nécessitant un masquage préalable de leur amertume, par des moyens, tels que l'enrobage polymérique, qui sont réputés particulièrement sensibles à la compression, et dont l'utilisation est incompatible avec l'application de forces de compression élevées, ce qui accroît le risque de rupture du film.

C'est pourquoi, à ce jour, parmi les formes solides destinées à se désintégrer dans la bouche, il n'existe de comprimé multicouche, que sous forme de comprimé ou pastille à sucer, pour l'administration de substances actives à action locale, limitée à la muqueuse buccale et à l'oropharynx et ne nécessitant aucun masquage de goût autre que la simple addition d'édulcorants.

Un exemple connu de tels comprimés pour une administration sublinguale est la Solutricine® vitamine C commercialisée en France par THERAPLIX qui est un comprimé triple couche comprenant de la tyrothrycine et de l'acide ascorbique.

Ces comprimés multicouches à sucer ont une dureté élevée pour assurer l'adhésion les couches, et présentent un temps de séjour dans la cavité buccale de plusieurs minutes, correspondant au temps pendant lequel le comprimé se désintègre progressivement.

L'érosion et la solubilisation, principaux mécanismes de désintégration du comprimé, dépendent alors directement de la taille du comprimé et de sa surface de contact avec la salive.

De part les contraintes qu'elles posent, les solutions proposées à ce jour pour formuler des associations de substances actives, ne peuvent donc pas s'appliquer à des comprimés orodispersibles, encore moins lorsque le goût des substances actives utilisées doit être masqué.

Il existe donc un réel besoin en des comprimés orodispersibles permettant l'association de différentes substances actives, éventuellement enrobées, sans présenter les inconvénients d'hétérogénéité de teneur ou d'incompatibilité.

La Demanderesse a trouvé contre toute attente qu'il est possible de d'obtenir un comprimé orodispersible multicouche.

Ainsi, la présente invention porte sur un comprimé qui est orodispersible et qui est constitué d'au moins deux couches superposées et solidaires, deux desdites couches comprenant chacune au moins une substance active.

Dans sa plus large définition, la présente invention porte sur des comprimés tels que définis dans les revendications attachés.

Chacune des couches comprend un mélange d'excipients de compression. Le mélange d'excipients comprend :
- au moins un agent soluble et
- au moins un agent désintégrant et/ou au moins un agent gonflant.

Le nombre de couches est limité par l'épaisseur résultante du comprimé qui doit être acceptable pour le patient et, de façon générale ne dépasse pas trois.

Dans un première variante de l'invention le comprimé orodispersible est un comprimé bicouche qui comprend au moins un substance active dans chaque couche.

Dans une seconde variante de l'invention, le comprimé orodispersible est un comprimé tricouche.

Dans ce cas, les trois couches peuvent contenir une substance active ou bien l'une des couches peut ne contenir que des excipients.

De façon avantageuse, la couche ne contenant que des excipients est intercalée entre les deux couches comprenant chacune au moins une substance active.

Selon une variante de l'invention, la substance active de deux des couches est une même molécule de base, mais diffère par la nature du sel ou de la base utilisée, ou bien par son état cristallin polymorphe ou amorphe, la solubilité et/ou les caractéristiques pharmacocinétiques de la molécule présente dans l'une des couches étant différentes de celles de la molécule présente dans une autre couche.

Selon une autre variante de l'invention, la substance active présente dans chacune des couches est chimiquement identique, mais est mise en forme de façon différente dans chacune des couches, de façon à présenter des vitesses de libération *in-vitro* et *in-vivo* significativement différentes.

La substance active se présente par exemple sous la forme de particules ayant des propriétés de libération modifiée, par exemple prolongée, de façon à libérer de façon efficace sur une période comprise entre 8 à 24 heures, ou retardée permettant de libérer la substance active sur un site d'absorption spécifique ou d'éviter sa dégradation dans un milieu de pH défavorable.

Dans cette variante, la substance active de l'autre couche se présente sous une forme immédiate, éventuellement enrobée si la molécule à besoin d'un simple masquage de goût, ou modifiée selon un profil de libération différent de la première couche.

Ces caractéristiques de libération ou de masquage de goût peuvent être atteintes par toute méthode connue permettant d'atteindre ce résultat, mais de façon préférée au moyen d'un enrobage polymérique autour de la particule de substance active.

Le profil plasmatique résultant de l'administration d'un tel comprimé chez un patient, présente plusieurs pics de concentrations plasmatiques, correspondant aux vitesses de libération différentes des particules de chaque couche, lesdites particules ayant été avalées simultanément, après désintégration du comprimé orodispersible.

La ou les substances actives peuvent être choisies dans toute famille de composés, par exemple parmi les sédatifs gastrointestinaux, les antiacides, les antalgiques, les antiinflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste ou tout autre famille de composés, les substances actives associées dans le comprimé pouvant être choisies dans la même famille ou dans des familles différentes.

Les substances actives peuvent se présenter sous la forme de leurs sels pharmaceutiquement acceptables ou toute forme polymorphe (racémique, énantiomère,...). Par "sels pharmaceutiquement acceptables", on entend les dérivés des composés décrits dans lesquels le composé pharmaceutiquement actif de base est transformé en son sel basique ou acide, des exemples de sels pharmaceutiquement actifs comprennent notamment les sels d'acides organiques ou minéraux de résidus basiques tels que les amines ; les dérivés alcalins ou les sels organiques de résidus acides tels que les acides carboxyliques, et similaires. Les sels pharmaceutiquement acceptables comprennent les sels non toxiques classiques ou les sels d'ammonium quaternaire du composé de base, formés par exemple à partir d'acides inorganiques ou organiques non toxiques. Par exemple, de tels sels non toxiques classiques comprennent ceux issus d'acides inorganiques tels que chlorhydrique. bromhydrique, sulfurique, sulfonique, sulfamique, phosphorique, nitrique et similaires ; et les sels préparés à partir d'acides organiques tels que les acides aminés, acétique, propionique, succinique, glycolique, stéarique, lactique, malique, tartrique, citrique, ascorbique, pamoïque, maléique, hydroxymaléique, phénylacétique, glutamique, benzoïque, salicylique, sulfanilique, 2-acétoxybenzoïque, fumarique, toluènesulfonique, méthanesulfonique, éthane disulfonique, oxalique, isothionique, et similaires.

Les sels pharmaceutiquement acceptables de la présente invention peuvent être synthétisés à partir du composé thérapeutique de base qui contient une fraction acide ou basique, par des procédés classiques. De façon générale, ces sels peuvent être préparés par réaction des formes acides ou basiques libres avec une quantité prédéterminée de la base ou de l'acide approprié dans l'eau ou dans un solvant organique ou dans un mélange d'eau et de solvant organique.

On préfère généralement des milieux non aqueux. Les listes de sels appropriés sont répertoriées dans Remington's Pharmaceutical Sciences, 17th ed., Mack Publishing Company, Easton, PA, 1985, p. 1418.

L'expression "pharmaceutiquement acceptable" est employée ici pour faire référence aux composés, matières, compositions et/ou formes galéniques qui sont, selon l'appréciation médicale, appropriés pour une utilisation au contact de tissus humains ou animaux sans toxicité, irritation, réponse allergique ou autre problème ou complication excessifs, pour un rapport bénéfice/risque raisonnable.

Le comprimé orodispersible multicouche selon l'invention est particulièrement adapté pour administrer des médicaments en association car il permet à la fois de diminuer le nombre d'unités à prendre chaque jour par le patient et d'améliorer l'observance des traitements chez les personnes ayant des difficultés à avaler.

Les associations sont particulièrement étudiées par les laboratoires pharmaceutiques, celles citées ci-dessous le sont à titre d'exemple sans aucun caractère limitant.

Les associations de principes actifs sont particulièrement utiles dans le domaine de l'antalgie, quand un effet de synergie est recherché, en combinant par exemple la morphine, l'oxycodone, l'hydrocodone ou le tramadol, avec un second antalgique tel que l'ibuprofène ou le paracétamol ou dans le domaine des anti-inflammatoires, en combinant le kétoprofène et le naproxène, ou le diclofenac avec le misoprostol.

Il est également possible d'administrer conjointement un analgésique opioïde, par exemple l'oxycodone ou la morphine avec un antagoniste des récepteurs opioïdes, tel que la naloxone ou la naltrexone, pour éviter la mauvaise utilisation du médicament par des toxicomanes.

Dans le domaine des antiulcéreux, les associations préférées combinent des agents antiulcéreux, par exemple un inhibiteur de la pompe à protons tel que l'oméprazole ou le lansoprazole, un inhibiteur des récepteurs H-2 tel que la famotidine ou la ranitidine ou encore un agent antiacide.

Dans le domaine des hypocholestérolémiants et antidiabétiques, il est possible de combiner entre elles des molécules appartenant à des familles différentes, parmi lesquelles les fibrates, par exemple le fénofibrate, les biguanides, telles que la metformine, ou les statines, telles que l'atorvastatine ou la simvastatine.

D'autres domaines sont particulièrement étudiés tels que ceux des médicaments efficaces contre le virus du SIDA ou les anticancéreux.

La substance active, dont la taille peut-être comprise entre 20µm et 1000µm, peut se présenter sous la forme de poudre ou de microcristaux, ou sous la forme de granulés obtenus par granulation, sèche, humide ou à chaud, ou bien encore sous la forme de granules obtenus par montage sur supports neutres, ou extrusion-sphéronisation.

Dans la suite de la description, le terme « particule active » sera utilisé pour désigner indifféremment l'une ou l'autre de ces formes sous lesquelles peut être mise en oeuvre la substance active.

La substance active, initialement sous forme de poudre ou de microcristaux, est utilisée à l'état sec pour la granulation, et sous forme de solution ou suspension dans un solvant aqueux ou organique pour le montage sur supports inertes.

Le support inerte peut être constitué par tout excipient inerte chimiquement et pharmaceutiquement, existant sous forme particulaire, cristalline ou amorphe, par exemple des dérivés de sucres tels que le lactose, le saccharose, l'amidon hydrolysé (maltodextrines) ou encore des celluloses.

Des mélanges tels que le saccharose et l'amidon, ou à base de cellulose sont également utilisés pour la préparation de supports inertes sphériques.

La dimension particulaire unitaire du support inerte peut être comprise entre 50µm et 500µm, de préférence entre 90µm et 150µm.

La particule active peut en outre comprendre un ou plusieurs excipients choisis dans le groupe comprenant les agents liants, les diluants, les agents antistatiques, les agents modifiant le micro-pH environnant et leurs mélanges.

L'agent liant est présent dans des proportions pouvant aller jusqu'à 15% en poids, de préférence jusqu'à 10% en poids par rapport au poids des particules non enrobées et peut être choisi dans le groupe comprenant notamment des polymères cellulosiques, des polymères acryliques, des povidones, des copovidones, des polyvinylalcools, l'acide alginique, l'alginate de sodium, l'amidon, l'amidon prégélatinisé, les sucroses et leurs dérivés, la gomme guar, les polyéthylèneglycols et leurs mélanges.

Le diluant est présent dans des proportions pouvant aller jusqu'à 95% en poids, de préférence jusqu'à 50% en poids par rapport au poids des particules non enrobées et peut être choisi dans le groupe comprenant notamment les dérivés cellulosiques et préférentiellement la cellulose microcristalline, les polyols et préférentiellement le mannitol, les amidons seuls, les dérivés de sucres tels que le lactose, et leurs mélanges.

L'agent antistatique est présent dans des proportions pouvant aller jusqu'à 10% en poids, de préférence jusqu'à 3% en poids par rapport au poids des particules non enrobées et peut être choisi dans le groupe comprenant notamment la silice colloïdale, notamment celle commercialisée sous la marque Aerosil®, et préférentiellement la silice précipitée, notamment celle commercialisée sous le nom Syloïd^{®} FP244, le talc micronisé ou non, et leurs mélanges.

L'agent modifiant le micro-pH environnant peut être un composé acide ou alcalin.

L'agent acide peut être constitué par tout acide minéral ou organique, sous forme d'acide libre, d'anhydride d'acide ou de sel d'acide.

Cet acide est choisi dans le groupe comprenant notamment l'acide tartrique, l'acide citrique, l'acide maléique, l'acide fumarique, l'acide malique, l'acide adipique, l'acide succinique, l'acide lactique, l'acide glycolique, les alpha hydroxyacides, l'acide ascorbique et les acides aminés, ainsi que les sels et dérivés de ces acides.

Le composé alcalin est choisi dans le groupe comprenant le carbonate de potassium, de lithium, de sodium, de calcium, d'ammonium ou le carbonate de L-lysine, le carbonate d'arginine, le carbonate de glycine sodique, les carbonates sodiques d'acides aminés, le perborate de sodium anhydre, le perborate effervescent, le monohydrate de perborate sodique, le percarbonate de sodium, le dichloroisocyanurate de sodium, l'hypochlorite de sodium, l'hypochlorite de calcium, et leurs mélanges.

Dans le cadre de la présente invention, le carbonate est indifféremment un carbonate, un sesquicarbonate ou un hydrogénocarbonate.

La quantité d'agent modifiant le micro-pH environnant est comprise entre 0,5 et 20% en poids, par rapport au poids des particules non enrobées, de préférence entre 5 et 15%, et de façon encore préférée entre 5 et 10% en poids par rapport au poids des particules non enrobées.

Le cas échéant, la poudre, les microcristaux ou les particules de substance active peuvent avantageusement être enrobés d'une couche fonctionnelle dont la composition est choisie en fonction des caractéristiques souhaitées, notamment de masquage de goût et/ou de libération modifiée, retardée ou prolongée.

La composition de l'enrobage est choisie en fonction des caractéristiques physico-chimiques de chaque substance active et est constituée d'au moins un polymère d'enrobage.

Le polymère d'enrobage peut être insoluble ou soluble seulement à certaines valeurs de pH, et il est choisi avantageusement dans le groupe comprenant les polymères cellulosiques, les polymères acryliques, les polymères vinyliques et leurs mélanges.

Parmi les polymères cellulosiques, on choisira avantageusement l'éthylcellulose, l'hydroxypropylcellulose (HPC) et l'hydroxypropylméthylcellulose (HPMC), la cellulose acétate, la cellulose acétatophtalate, l'hydroxy-propylméthylcellulose phtalate, l'hydroxypropyl-méthylcellulose succinate phtalate, la cellulose acétate, la cellulose acétate trimellitate, la cellulose acétate butyrate, la carboxyméthylcellulose, seuls ou en mélange.

Parmi les polymères acryliques, on choisira avantageusement le copolymère ammonio-méthacrylate (Eudragit^{®} RL et RS), le polyacrylate (Eudragit^{®} NE) et le polyméthacrylate (Eudragit^{®} E), le copolymère d'acide méthacrylique, commercialisé sous le nom de marque Eudragit® L100 ou Eudragit® L30D, Eudragit^{®} étant une marque déposée par RÖHM.

D'autres polymères sont par exemple le shellac, le polyvinyl acétate phtalate, ou tout autre polymère, utilisés seuls, en mélange, ou combinés séparément.

La composition d'enrobage est appliquée de préférence par pulvérisation d'une solution, d'une suspension ou encore d'une dispersion colloïdale du polymère d'enrobage dans un solvant ou un mélange de solvants, pour former un film continu recouvrant la totalité de la surface de chaque particule, et ce, quel que soit son état de surface, en quantité suffisante pour permettre d'obtenir, par exemple, un masquage de goût efficace au moment de la prise du médicament et pendant tout le temps de séjour des particules enrobées dans la cavité buccale.

L'épaisseur du film, qui est généralement comprise entre 5 µm et 75 µm, dépend le plus souvent de la solubilité de la substance active au pH de la salive et du caractère plus ou moins prononcé de son amertume.

Le polymère est appliqué à la surface des particules de substances actives dans des proportions pouvant aller jusqu'à 60%, de préférence jusqu'à 20%, calculé en gain de poids par rapport à la masse de particules enrobées.

Le solvant choisi pour pulvériser le polymère d'enrobage peut être l'eau, un solvant organique, tel que l'éthanol, l'isopropanol, l'acétone, le chlorure de méthylène ou un mélange de solvants.

La composition d'enrobage comprend également de façon optionnelle, un plastifiant, un agent tensioactif, un agent antistatique et/ou un lubrifiant.

Le plastifiant est utilisé dans une proportion d'au plus 40%, de préférence entre 15 à 30%, exprimé en poids par rapport au poids sec de polymère et choisi dans le groupe comprenant le triéthyl citrate, l'acétyltributyl citrate, la triacétine, le tributyl citrate, le diéthylphtalate, les polyéthylènes glycols, les polysorbates, les glycérides mono-et diacétylés, et leurs mélanges.

L'agent tensioactif est choisi parmi les tensioactifs anioniques, cationiques, non ioniques ou amphotères.

L'agent antistatique est utilisé dans une proportion d'au plus 10% en poids, de préférence comprise entre 0 et 3% en poids, de préférence inférieure à 1% en poids, calculé par rapport au poids sec du polymère, dans le groupe comprenant le talc micronisé ou non micronisé, la silice colloïdale (Aerosil®200), la silice traitée (Aerosil®R972), ou la silice précipitée (Syloïd® FP244) et leurs mélanges.

Le lubrifiant est utilisé dans une proportion d'au plus 10% en poids, de préférence entre 0 et 3% et de façon encore préférée, inférieure à 1% en poids, calculé par rapport au poids sec du polymère et est choisi dans le groupe comprenant le stéarate de magnésium, l'acide stéarique, le sodium stéaryl fumarate, les polyoxyéthylèneglycols, le benzoate de sodium et leurs mélanges.

La taille des particules enrobées est habituellement comprise entre 50µm et 1000µm, de préférence entre 100µm et 800µm, de façon encore préférée entre 200 et 500µm, et est déterminée par les méthodes conventionnelles, par exemple à l'aide d'un jeu de tamis d'ouverture de mailles calibrées, ou par diffraction d'un laser.

La distribution granulométrique habituellement préférée des particules enrobées, déterminée par l'une des méthodes ci-dessus, est telle qu'au moins 80% en poids de la population de particules enrobées à une taille comprise entre 90µm et 500 µm, et de façon encore préférée entre 150µm et 500 µm, et avec une valeur de D_{50%} comprise entre 200 et 400 µm.

Le mélange d'excipients présent dans chacune des couches du comprimé est parfois appelé dans la suite de la description « excipients de compression » par opposition aux excipients utilisés pour la mise en forme des particules de substance active.

Ce mélange comprend nécessairement au moins un agent soluble, au moins un agent désintégrant et/ou au moins un agent gonflant.

L'agent soluble est choisi parmi les sucres tels que le saccharose, le lactose, le fructose, le dextrose, ou les polyols de moins de 13 atomes de carbone tels que le mannitol, le xylitol, le sorbitol, le maltitol, le lactitol, l'érythritol, seuls ou en mélange.

L'agent soluble est utilisé dans une proportion comprise entre 20 et 90% en poids, de préférence entre 30 et 60% en poids, calculée par rapport au poids de chaque couche du comprimé.

L'agent soluble est utilisé sous sa forme directement compressible, dont le diamètre moyen des particules est de 100µm à 500µm, où sous la forme d'une poudre dont le diamètre moyen des particules est inférieur à 100 µm, ladite poudre étant utilisée seule ou en mélange avec le produit directement compressible.

Chaque couche du comprimé peut comprendre un seul agent soluble ou un mélange d'au moins deux agents solubles, l'agent soluble pouvant dans tous les cas être indifféremment utilisé sous sa forme directement compressible ou sous forme de poudre non directement compressible.

Le comprimé peut comprendre le même agent soluble dans chacune des couches ou le même mélange d'agents solubles, mais la composition peut également varier d'une couche à l'autre, aussi bien en ce qui concerne la nature de l'agent soluble, la taille des particules de celui-ci, ou, dans le cas d'un mélange, le ratio de chacune des fractions.

Dans un premier mode de réalisation avantageux du comprimé de l'invention, chaque couche du comprimé contient un seul agent soluble utilisé sous sa forme directement compressible.

Dans un second mode de réalisation avantageux du comprimé de l'invention, chaque couche du comprimé contient un mélange comprenant un agent soluble sous sa forme directement compressible et le même agent soluble sous la forme de poudre, les proportions respectives de la forme directement compressible et de la poudre étant comprises entre 99/1 et 20/80, de préférence entre 80/20 et 20/80.

Dans un troisième mode de réalisation avantageux du comprimé de l'invention, le comprimé contient le même agent soluble ou le même mélange d'agents solubles dans chacune des couches qui le compose.

L'agent de désintégration est choisi dans le groupe comprenant notamment la carboxyméthylcellulose sodique réticulée désignée dans le métier par le terme croscarmellose, les polyvinylpyrrolidones réticulées désignées dans le métier par le terme crospovidones, et leurs mélanges.

L'agent de désintégration est utilisé dans une proportion comprise entre 1 et 20% en poids, de préférence entre 5 et 15% en poids, dans le cas d'un mélange, chaque agent de désintégration étant compris entre 0,5 et 15% en poids, de préférence entre 5 et 10% en poids, calculée par rapport poids de chaque couche du comprimé.

L'agent gonflant est choisi dans le groupe comprenant la cellulose microcristalline, les amidons, les amidons modifiés, tels que le carboxyméthylamidon ou le glycolateamidon sodique, l'acide alginique ou l'alginate de sodium, et leurs mélanges.

L'agent gonflant est utilisé dans une proportion comprise entre 1 et 15% en poids calculée par rapport au poids de chaque couche du comprimé.

Outre les excipients cités précédemment, chaque couche du comprimé orodispersible de l'invention peut comprendre de façon optionnelle, un lubrifiant, un agent perméabilisant, un agent antistatique, un diluant insoluble dans l'eau, un liant, un édulcorant, un arôme, un colorant, des adjuvants.

Le lubrifiant est choisi dans le groupe comprenant le stéarate de magnésium, l'acide stéarique, le sodium stéaryl fumarate, les polyoxyéthylèneglycols, le benzoate de sodium, une huile pharmaceutiquement acceptable, de préférence la diméthicone ou la paraffine liquide, et leurs mélanges.

Le lubrifiant est utilisé dans une proportion pouvant aller jusqu'à 2% en poids, de préférence comprise entre 0,02 et 2% en poids, de façon davantage préférée comprise entre 0,5 et 1% en poids, calculée par rapport au poids de chaque couche du comprimé.

Dans une première variante, le lubrifiant est incorporé en totalité au mélange d'excipients de compression, dans une seconde variante, au moins une fraction de ce lubrifiant est pulvérisée sur les parois de la matrice et des poinçons au moment de la compression, ladite fonction de lubrifiant étant alors sous la forme d'une poudre, ou d'un liquide.

Les quantités de lubrifiant utilisées en phase interne et/ou externe sont ajustées avec soin de façon à éviter qu'un excès n'altère la cohésion des couches au moment de la compression finale.

L'agent perméabilisant est choisi dans le groupe comprenant notamment des silices ayant une grande affinité pour les solvants aqueux, telles que la silice précipitée plus connue sous le nom de marque Syloïd^{®}, les maltodextrines, les β-cyclodextrines et leurs mélanges.

L'agent perméabilisant est utilisé dans une proportion pouvant aller jusqu'à 5% en poids, calculée par rapport au poids de chaque couche du comprimé.

L'agent antistatique peut être choisi dans le groupe comprenant le talc micronisé ou non micronisé, la silice colloïdale (Aerosil®200), la silice traitée (Aerosil®R972), ou la silice précipitée (Syloïd® FP244) et leurs mélanges.

L'agent antistatique est utilisé dans une proportion pouvant aller jusqu'à 5% en poids, calculée par rapport au poids de chaque couche du comprimé.

Le diluant insoluble dans l'eau peut être choisi parmi le phosphate dicalcique, le phosphate tricalcique ou une cellulose microcristalline.

Son rôle est d'améliorer l'action de l'agent désintégrant en augmentant la charge non soluble dans le comprimé. Il est utilisé dans une proportion pouvant aller jusqu'à 20% en poids, de préférence inférieure à 10% en poids, calculée par rapport au poids de chaque couche du comprimé.

Le liant est utilisé sous forme sèche et peut être un amidon, un sucre, la polyvinylpyrrolidone ou la carboxyméthylcellulose, seuls ou en mélange.

Il est utilisé de préférence dans une seule des couches du comprimé et dans une proportion pouvant aller jusqu'à 15% en poids, de préférence inférieure à 10% en poids, calculée par rapport au poids de la couche dans laquelle il se trouve.

L'édulcorant peut être choisi dans le groupe comprenant notamment l'aspartam, l'acésulfame de potassium, le saccharinate de sodium, la néohespéridine dihydrochalcone, le sucralose, le monoammonium glycyrrhizinate, et leurs mélanges.

Les arômes et les colorants sont ceux utilisés habituellement en pharmacie pour la préparation de comprimés.

Dans un mode de réalisation particulièrement préféré, chaque couche présente une coloration différente de celle à laquelle elle est accolée, de telle sorte que la structure en couche du comprimé soit immédiatement visible.

Des adjuvants peuvent également être ajoutés au mélange, et sont choisis dans le groupe comprenant les accélérateurs de désintégration, par exemple des acides aminés ou des protéines, les agents ajusteurs de pH, les systèmes permettant de produire une effervescence, notamment générateurs de dioxyde de carbone du type de ceux utilisés comme agents ajusteurs de pH, ou encore les tensioactifs.

Dans une couche comprenant une substance pharmaceutiquement active, la proportion de mélange d'excipients par rapport à la substance active, enrobée ou non, est habituellement comprise entre 0,4 et 10 de préférence entre 1 à 5 parties en poids.

Dans un mode de réalisation avantageux du comprimé de l'invention, chaque couche du comprimé comprend les mêmes excipients de façon à ce que la désintégration du comprimé de l'invention procure une sensation en bouche qui soit identique à celle que procure un comprimé orodispersible « monocouche » de même composition qualitative, et que le patient ne perçoive aucune différence de vitesse de désintégration entre les différentes couches constituant le comprimé.

La composition quantitative de chaque couche, elle, est ajustée pour tenir compte des teneurs de chaque substance active.

Le rapport maximal de masse toléré entre la couche la plus épaisse et la couche la moins épaisse est de 10/1.

Dans le cas où le rapport de dose entre la substance active la plus dosée et la moins dosée est supérieur à 10, la quantité de diluant est ajustée de telle sorte que le rapport pondéral entre les couches soit ramené à une valeur de 10. Le diluant est dans ce cas de préférence un agent soluble, de façon encore préférée un agent soluble sous une forme directement compressible.

Les comprimés peuvent avoir un diamètre compris entre 6 mm et 18 mm.

Leur forme peut être ronde, ovale, oblongue, présenter une surface plate, concave ou convexe, et éventuellement présenté des gravures.

Des poinçons de forme biconvexes sont avantageusement utilisés.

Les comprimés ont de façon générale une masse comprise entre 0,1 gramme et 2,0 grammes.

L'invention porte également sur le procédé de préparation des comprimés multicouches précédemment décrits.

Le procédé conforme à l'invention comprend les étapes suivantes :
1. préparation d'au moins deux types de particules de substances actives éventuellement enrobées ;
2. préparation d'au moins deux mélanges secs comprenant chacun des excipients de compression et au moins un type de particules de substance active ;
3. pré-compression d'au moins un des mélanges de poudres obtenu ci-dessus ;
4. application sur le mélange ci-dessus d'un autre mélange ;
5. pré-compression éventuelle ;
6. compression finale sur les couches pré-formées obtenues précédemment,
les étapes 4 et 5 pouvant être répétées au moins une fois selon le nombre de couches du comprimé.

Dans le cas d'un comprimé bicouche, le procédé conforme à l'invention comprend les étapes suivantes :
- préparation de deux types de particules de substance active, éventuellement enrobées ;
- préparation de deux mélanges à sec comprenant chacun les excipients de compression et les particules de substance active préparées ci-dessus,
- pré-compression de l'un des mélanges ci-dessus de façon à préformer la couche inférieure du comprimé,
- application sur la couche préformée du second mélange ;
- éventuellement, pré-compression du second mélange de façon à préformer la couche supérieure du comprimé,
- compression finale.

Dans le cas d'un comprimé tricouche, le procédé conforme à l'invention comprend les étapes suivantes :
- préparation d'au moins deux types de particules de substance active, éventuellement enrobées ;
- préparation de trois mélanges à sec comprenant chacun les excipients de compression et dont au moins deux comprennent en outre les particules de substance active préparées ci-dessus,
- pré-compression de l'un des mélanges ci-dessus de façon à préformer la couche inférieure du comprimé,
- application sur la couche préformée d'un second mélange ;
- pré-compression du second mélange de façon à préformer la couche intermédiaire du comprimé,
- application sur la couche préformée du troisième mélange ;
- éventuellement, pré-compression du troisième mélange de façon à préformer la couche supérieure du comprimé,
- compression finale.

Dans un mode d'obtention préféré, la préparation de chaque mélange comprend elle-même deux étapes, la première étape consistant à mélanger la substance active, enrobée ou non, avec tous les excipients de compression sauf le lubrifiant interne, puis une seconde étape, dans laquelle le lubrifiant est ajouté au premier mélange en totalité ou en partie, la partie restante étant alors pulvérisée sur les poinçons et /ou sur la face interne des matrices de compression.

Lorsque la totalité du lubrifiant est pulvérisée sur les poinçons et /ou sur la face interne des matrices de compression la deuxième étape du mélange est alors bien entendu supprimée.

Les étapes de pré-compression et de compression sont réalisées sur une machine à comprimer alternative ou rotative.

La pré-compression vise d'une part à pré-former la couche en tassant le lit de poudre dans la matrice de compression, et d'autre part à dégazer ledit lit de poudre, en réorganisant les particules, de façon à éviter l'apparition de clivage au moment de la compression finale, clivage pouvant intervenir soit entre les couches, par défaut d'adhésion, soit dans la couche elle-même.

Dans un comprimé dont les couches n'auraient pas la même importance relative en masse et/ou en épaisseur, la première couche pré-formée est celle dont la masse ou l'épaisseur est la plus importante.

Les contraintes exercées lors de l'étape de pré-compression peuvent varier de 0,5 à 5kN, et sont en général 5 à 10 fois inférieures aux contraintes exercées lors de la compression finale.

Les contraintes exercées lors de l'étape de compression peuvent varier de 5kN à 50kN, de préférence de 5 kN à 15 kN.

La régulation des forces de pré-compression appliquées sur les lits de poudres intervient selon deux modes possibles, le premier consiste à régler la force de compression en fonction des variations mesurées par la machine au niveau des hauteurs du lit de poudre dans la matrice, le second consiste à régler le volume de remplissage en fonction de la pression mesurée exercée par les poinçons.

La dureté de ces comprimés est comprise entre 1 et 6kp, mesurée selon la méthode de la Pharmacopée Européenne (2.9.8), 1kp étant égal à 9,8N.

La dureté du comprimé multicouche est adaptée de façon à obtenir une friabilité, mesurée selon la méthode de la Pharmacopée Européenne, inférieure à 1% en poids, et à permettre un temps de désintégration du comprimé dans la bouche sous l'action de la salive, inférieur à 60 secondes, de préférence inférieur ou égal à 40 secondes.

Dans le cas où le comprimé de l'invention contient une substance active sous une forme enrobée, que ce soit pour en masquer le goût ou pour retarder ou prolonger la libération, la compression doit être réalisée de façon à conserver un profil de dissolution identique entre les particules de substances actives enrobées avant et après compression, identique devant être compris comme ne différant pas de plus de 15% en valeur absolue par rapport au pourcentage de substance active libérée à chaque temps de prélèvement dans les mêmes conditions de dissolution *in-vitro.*

L'invention sera mieux comprise au moyen des exemples de réalisation des comprimés conformes à l'invention. Ces exemples sont donnés uniquement à titre d'illustration et de modes de réalisation avantageux de l'invention et n'en constituent nullement une limitation.

### Excipients utilisés

Mannitol M 300 directement compressible : PARTECK® commercialisé par MERCK
Mannitol 60 poudre : Pearlitol® 160C commercialisé par Roquette Frères
Crospovidone : Kollidon® CL commercialisé par BASF
Sucralose : commercialisé par McNeill
Aspartame : commercialisé par Nutrasweet
Arôme Rootbeer mint et arôme biscuit vanilla : commercialisés par Pharmarôme
Stéarate de magnésium : commercialisé par Peter Graven.

### Equipement

Le mélangeur est un mélangeur par retournement de marque SONECO ou BSI de 60 1 ou 200 L.

La machine à comprimer utilisée dans les exemples 1, 2 et 3 est une presse COURTOY R292F équipée de 55 stations de type B, dont seules 28 stations ont été utilisées.

La machine comprend un système de double alimentation et peut être utilisée en double sortie lors d'une compression à grande vitesse de comprimés monocouche ou en simple sortie lors de la fabrication de comprimés bicouche.

La machine à comprimer utilisée dans les exemple 4 et 5 est une presse FETTE PT3090 équipée de 61 stations de type B et 49 stations de type D.

### EXEMPLE 1 : Comprimé orodispersible bicouche contenant 500 mg de paracétamol et 65 mg de caféine.

### 1/ Mélanges

Le premier mélange de poudres (couche A) est préparé selon la formule du tableau 1.

**Tableau 1**

| | FORMULA (%p/p) |
|---|---|
| PARACETAMOL ENROBE | 46,9 |
| MANNITOL M300 | 21,5 |
| MANNITOL 60 | 21,5 |
| KOLLIDON CL | 6,9 |
| SUCRALOSE | 1,0 |
| ROOTBEER MINT FLAVOR | 1,0 |
| BISCUIT VANILLA FLAVOR | 0,2 |
| MAGNESIUM STEARATE | 1,0 |
| TOTAL | 100 |

Les particules enrobées de paracétamol sont obtenues par granulation et enrobage en lit d'air fluidisé.

La distribution granulométrique desdites particules est déterminée par diffraction laser et présente les caractéristiques suivantes :
98% en poids des particules enrobées ont une taille comprise entre 150µm et 500 µm.

Un pré-mélange arômatisé constitué du mannitol 60, du Kollidon CL, du sucralose et des arômes est préparé par mélange des différents ingrédients dans les proportions données dans le Tableau 1, pendant 15 min à 10 tours par minute.

A ce premier mélange est ajouté le mannitol M300 et les granulés de paracétamol enrobé dans les proportions données dans le Tableau 1.

Le temps de mélange est de 20 minutes à la vitesse de 10 tours/minute.

Le lubrifiant est ajouté au mélange ainsi obtenu par mélange (étape de lubrification) pendant 2 minutes à la vitesse de 10 tours/minute.

Le second mélange, comprenant la caféine enrobée et les excipients de compression donnés dans le Tableau 2, est préparé strictement selon le même protocole que celui décrit ci-dessus pour le premier mélange.

**Tableau 2**

| | FORMULA (%p/p) |
|---|---|
| CAFEINE ENROBEE | 42,3 |
| MANNITOL M300 | 23,2 |
| MANNITOL 60 | 23,2 |
| KOLLIDON CL | 7,4 |
| SUCRALOSE | 1,1 |
| ROOTBEER MINT FLAVOR | 1,1 |
| BISCUIT VANILLA FLAVOR | 0,2 |
| GREEN COLOR | 0,5 |
| MAGNESIUM STEARATE | 1,0 |
| TOTAL | 100 |

Les particules enrobées de caféine sont également obtenues par granulation et enrobage en lit d'air fluidisé.

La distribution granulométrique desdites particules, déterminée par diffraction laser, présente les caractéristiques suivantes :
96% en poids des particules enrobées ont une taille comprise entre 150µm et 500 µm.

### 2/ Compression

La machine à comprimer est une presse COURTOY R292F équipée de 55 stations de type B, dont seules 28 stations ont été utilisées.

La première couche A (masse de 1200 mg) est tassée sous une force de pré-compression de 4,8 kN, l'épaisseur est déterminée pour obtenir une masse de 1200 mg.

Le mélange B (masse de 200 mg) est ensuite introduit dans la matrice à la surface de la couche A.

Une pré-compression de 2,3 kN est appliquée, avant la compression finale des deux couches successivement formées sous une force de 15,3 kN, pour cibler une dureté de 50 à 60 N.

Les poinçons utilisés sont ronds, plats et chanfreinés, de diamètre 16,5 mm.

Les comprimés bicouches ainsi préparés ont une masse théorique de 1400 mg et sont dosés à 500 mg de paracétamol et 65 mg de caféine.

La formule finale de chaque comprimé est la suivante (tableau 3) :

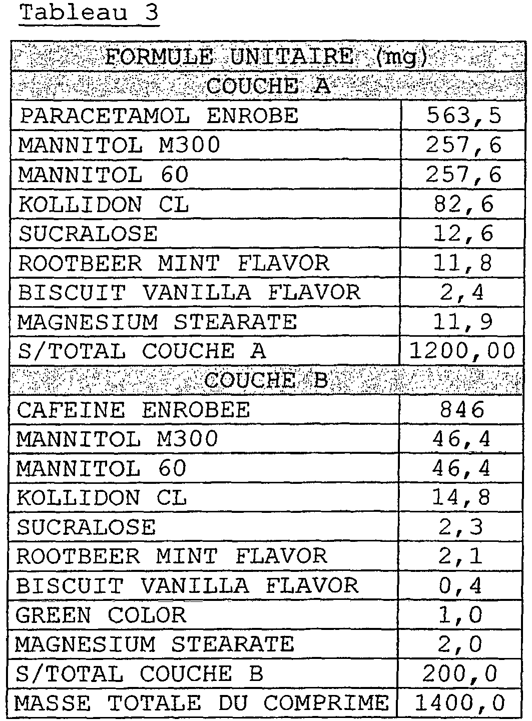

Ces comprimés ont les caractéristiques physiques et chimiques suivantes (tableau 4) :

**Tableau 4**

| | MOYENNE (CV) |
|---|---|
| Poids (mg) | 1400,1 |
| (n=16) | (2,7%) |
| Dureté(N) | 44,7 |
| (n=10) | (16,3%) |
| Désintégration en bouche | Min : 20 s |
| (n= 6) | Max : 35 s |

### EXEMPLE 2 : Comprimé orodispersible bicouche contenant 325 mg de paracétamol et 37,5 mg de chlorhydrate de tramadol (Tramadol HCl).

Un lot de 14000 comprimés bicouches est préparé de la façon suivante.

### 1/ Mélange

Tous les mélanges sont préparés selon le même protocole que l'exemple 1.

Le paracétamol enrobé présente des caractéristiques granulométriques identiques à celles de l'exemple 1.

Le premier mélange (COUCHE A, masse de 800 mg) comprend d'une part le paracétamol enrobé par 20% (calculé en poids d'enrobage par rapport au poids des particules enrobées) d'un mélange de polymères Eudragit^{®} E100/Eudragit^{®} NE30D dans un rapport 67/33, et d'autre part les excipients de compression dans les proportions données dans le tableau 5.

**Tableau 5**

| | FORMULE % (p/p) |
|---|---|
| PARACETAMOL ENROBE | 46,0 |
| MANNITOL M300 | 20,6 |
| MANNITOL 60 | 20,6 |
| KOLLIDON CL | 9,4 |
| ASPARTAME | 1,9 |
| ROOTBEER MINT FLAVOR | 0,9 |
| MAGNESIUM STEARATE | 0,6 |
| TOTAL | 100 |

Le second mélange (COUCHE B), comprend d'une part le chlorhydrate de tramadol enrobé par 35% (calculé en poids d'enrobage par rapport au poids des particules enrobées) d'éthylcellulose N7, et d'autre part les excipients de compression dans les proportions données dans le tableau 6.

Les particules enrobées de tramadol sont obtenues par granulation et enrobage en lit d'air fluidisé.

La distribution granulométrique desdites particules, déterminée par diffraction laser, présente les caractéristiques suivantes :

Des valeurs de D_{10%}, D_{50%}, et D_{90%} respectivement égales à 187 µm, 330 µm et 530 µm.

**Tableau 6**

| | FORMULE % (p/p) |
|---|---|
| TRAMADOL HCL ENROBE | 28,3 |
| MANNITOL M300 | 27,3 |
| MANNITOL 60 | 27,3 |
| KOLLIDON CL | 12,4 |
| ASPARTAM | 2,5 |
| ROOTBEER MINT FLAVOR | 1,2 |
| GREEN COLOR | 0,5 |
| MAGNESIUM STEARATE | 0,5 |
| TOTAL | 100 |

### 2/ Compression

La compression est réalisée à l'aide du même équipement que celui utilisé à l'exemple 1.

Le dosage moyen théorique de chaque comprimé est de 325 mg de paracétamol et 37,5 mg de tramadol HCl.

La machine à comprimer est équipée de poinçons ronds, plats et chanfreinés, de diamètre 15 mm.

La couche A (masse de 800 mg) est tassée sous une force de pré-compression de 1,6 kN.

Le mélange de poudres de la couche B (masse de 200mg) est ensuite introduit à la surface de la couche A prétassée.

Une force de pré-compression de 0,8 kN est appliquée, avant la compression finale des deux couches successivement formées, sous une force de 10 kN, pour cibler une dureté de 50N.

Dans ce lot de 14 000 comprimés, chaque comprimé a la composition finale suivante (tableau 7) :

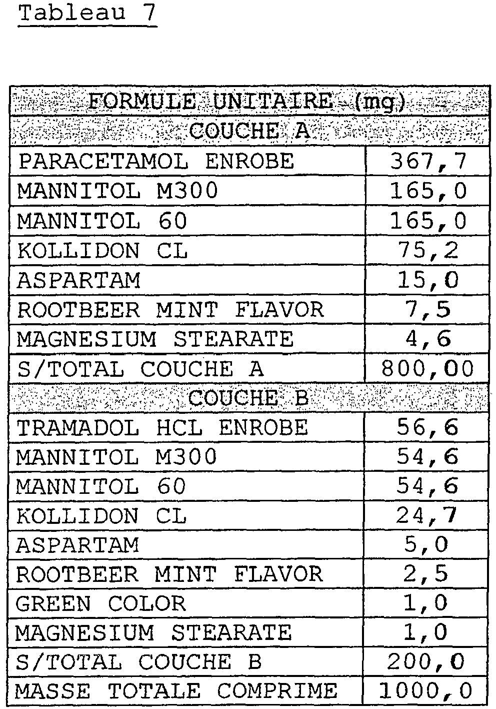

Ces comprimés ont les caractéristiques physiques et chimiques suivantes (tableau 8) :

**Tableau 8**

| | MOYENNE (CV) |
|---|---|
| Poids (mg) | 1005,1 |
| (n=16) | (0.42%) |
| Dureté (N) | 40.7 |
| (n=10) | (5.6%) |
| Désintégration in vitro | Min : 12 s |
| (n= 6) | Max : 28 s |
| Désintégration en bouche (n =3) | 20 à 35 s |
| Teneur en Paracétamol | 326, 7 |
| (n = 3) | (0,9%) |
| Teneur en tramadol | 41,7 |
| (n = 3) | (1,6) |

### EXEMPLE 3 : Comprimé orodispersible bicouche contenant 200 mg d'ibuprofène et 37,5 mg de chlorhydrate de tramadol (Tramadol HCl).

Un lot de 14000 comprimés bicouches est préparé de la façon suivante.

### 1/ Mélanges

Tous les mélanges sont préparés selon le même protocole que dans l'exemple 1.

Les particules enrobées d'ibuprofène sont obtenues par granulation et enrobage en lit d'air fluidisé.

La distribution granulométrique desdites particules, déterminée par diffraction laser, présente les caractéristiques suivantes :
Une valeur de D_{50%} égale à 258 µm, 2% en poids des particules ayant une taille inférieure à 90 µm et 1% en poids de ces mêmes particules ayant une taille supérieure à 500 µm.

Le premier mélange (COUCHE A) comprend d'une part l'ibuprofène enrobé par 13,7% (calculé en poids d'enrobage par rapport au poids des particules enrobées) d'éthylcellulose N7, et d'autre part et les excipients de compression dans les proportions données dans le tableau 9.

**Tableau 9**

| | FORMULE % (p/p) |
|---|---|
| IBUPROFENE ENROBE | 32,0 |
| MANNITOL M300 | 27,0 |
| MANNITOL 60 | 27,0 |
| KOLLIDON CL | 9,9 |
| ASPARTAM | 2,5 |
| ROOTBEER MINT FLAVOR | 1,0 |
| MAGNESIUM STEARATE | 0,6 |
| TOTAL | 100 |

Le second mélange (COUCHE B), comprend d'une part le chlorhydrate de tramadol enrobé par 35% (calculé en poids d'enrobage par rapport au poids des particules enrobées) d'éthylcellulose N7, et d'autre part et les excipients de compression dans les proportions indiquées dans le tableau 10.Les particules enrobées de tramadol ont des caractéristiques de taille identiques à celle de l'exemple 2.

**Tableau 10**

| | FORMULE % (p/p) |
|---|---|
| TRAMADOL HCL ENROBE | 28,3 |
| MANNITOL M300 | 28,4 |
| MANNITOL 60 | 28,4 |
| KOLLIDON CL | 10,4 |
| ASPARTAM | 2,6 |
| ROOTBEER MINT FLAVOR | 1,0 |
| GREEN COLOR | 0,5 |
| MAGNESIUM STEARATE | 0,4 |
| TOTAL | 100 |

### 2/ Compression

Le dosage moyen théorique est de 200 mg d'ibuprofène et 37,5 mg de tramadol HCl.

La machine à comprimer est équipée de poinçons ronds, plats et chanfreinés, de diamètre 15 mm.

La première couche A (masse de 800 mg) est tassée sous une force de pré-compression de 1,6 kN.

Le mélange de poudres de la couche B (masse de 200 mg) est ensuite introduit dans la matrice à la surface de la couche A pré-formée.

Une force de pré-compression de 0,8 kN est appliquée, avant la compression finale des deux couches successivement formées sous une force de compression de 10 à 12kN, avec pour cible une dureté de 50N.

Chaque comprimé a la composition finale suivante (tableau 11) :

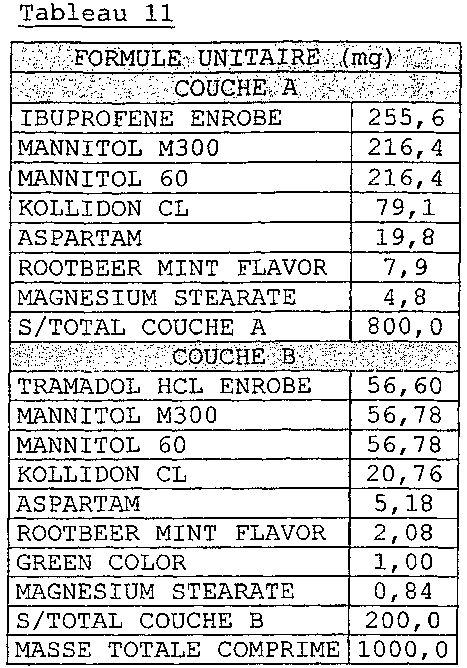

Ces comprimés ont les caractéristiques physiques et chimiques suivantes (tableau 12) :

**Tableau 12**

| | MOYENNE (CV) |
|---|---|
| Poids (mg) | 998, 5 |
| (n=20) | (0,4%) |
| Dureté (N) | 50,9 |
| (n=10) | (8,0%) |
| Désintégration in vitro | Min : 14 s |
| (n= 6) | Max : 20 s |
| Désintégration en bouche | 30 à 35 s |
| (n = 3) | |
| Teneur en ibuprofène | 205, 1 |
| (n = 3) | (0,6%) |
| Teneur en tramadol | 38,3 |
| (n = 3) | (0,3%) |

### EXEMPLE 4 : Comprimé orodispersible bicouche contenant 500 mg de paracétamol et 65 mg de caféine :

### 1/ Mélanges

Le premier mélange de poudres (couche A) est préparé selon la formule du tableau 13.

**Tableau 13**

| | FORMULE (% p/p) |
|---|---|
| PARACETAMOL ENROBÉ | 47,2 |
| MANNITOL M300 | 21,6 |
| MANNITOL 60 | 21,6 |
| KOLLIDON CL | 6,9 |
| SUCRALOSE | 1,1 |
| ROOTBEER MINT FLAVOUR | 1,0 |
| BISCUIT VANILLA FLAVOUR | 0,2 |
| STEARATE DE MAGNESIUM INTERNE | 0,4 |
| TOTAL | 100 |

Le second mélange comprend la caféine enrobée et les excipients de compression dans les proportions indiquées dans le tableau 14.

**Tableau 14**

| | FORMULE (% p/p) |
|---|---|
| CAFÉINE ENROBÉE | 42,5 |
| MANNITOL M300 | 23,3 |
| MANNITOL 60 | 23,3 |
| KOLLIDON CL | 7,5 |
| SUCRALOSE | 1,2 |
| ROOTBEER MINT FLAVOUR | 1,1 |
| BISCUIT VANILLA FLAVOUR | 0,2 |
| GREEN COLOUR | 0,5 |
| STEARATE DE MAGNESIUM | 0,4 |
| TOTAL | 100 |

Le deux mélanges sont préparés selon le même protocole que celui de l'exemple 1.

Les particules enrobées de paracétamol et de caféine ont les mêmes caractéristiques granulométriques que celles de l'exemple 1

### 2/ Compression

33 stations (parmi les 49 stations de type D de la machine à comprimer PT 3090) sont équipées de poinçons ronds en forme de fossettes de 17 mm de diamètre.

On utilise une lubrification externe de stéarate de magnésium pour lubrifier les poinçons et les matrices.

La première couche A (masse de 1800 g) est tassée sous une force de précompression de 2,2 kN, l'épaisseur étant déterminée pour avoir une masse de 1200 g.

Le mélange B (masse de 200 g) est alors introduit dans la matrice à la surface de la couche A.

Une précompression de 11,2 kN est appliquée avant la compression finale des deux couches successivement formées, sous une force de 15,3 kN, pour cibler une dureté de 70 N.

89 438 comprimés sont préparés avec une vitesse maximale de fabrication des comprimés de 80000 comprimés/heure.

Les comprimés ainsi préparés ont une masse théorique de 1400 mg et contiennent une dose de 500 mg de paracétamol et une dose de 65 mg de caféine.

La composition finale de chaque comprimé est comme suit :

**Tableau 15**

| Formule unitaire (mg) | |
|---|---|
| COUCHE A | |
| PARACETAMOL ENROBÉ | 556,9 |
| MANNITOL M300 | 259,2 |
| MANNITOL 60 | 259,2 |
| KOLLIDON CL | 83,0 |
| SUCRALOSE | 12,7 |
| ROOTBEER MINT FLAVOUR | 11,9 |
| BISCUIT VANILLA FLAVOUR | 2,4 |
| STEARATE DE MAGNESIUM | 4,7 |
| S/TOTAL COUCHE A | 1200,00 |

| COUCHE B | |
|---|---|
| CAFÉINE ENROBÉE | 84,6 |
| MANNITOL M300 | 46,4 |
| MANNITOL 60 | 46,4 |
| KOLLIDON CL | 14,8 |
| SUCRALOSE | 2,3 |
| ROOTBEER MINT FLAVOUR | 2,1 |
| BISCUIT VANILLA FLAVOUR | 0,4 |
| GREEN COLOUR | 1,0 |
| STEARATE DE MAGNESIUM | 2,0 |
| S/TOTAL COUCHE B | 200,00 |
| MASSE TOTALE DU COMPRIMÉ | 1400,00 |

Ces comprimés ont les caractéristiques physiques et chimiques suivantes (tableau 16) :

**Tableau 16**

| | MOYENNE (CV) |
|---|---|
| Poids (mg) | 1390,2 |
| (n = 20) | (1,9%) |
| Dureté (N) | 70,7 |
| (n = 10) | (5,4%) |
| Désintégration en bouche | 30 s |
| (n = 6) | |

### EXEMPLE 5 : Comprimé orodispersible bicouche contenant 325 mg de paracétamol et 37,5 mg de chlorohydrate de tramadol (tramadol HCl) :

### 1/ Mélange

Tous les mélanges sont préparés conformément à la première étape de l'exemple 2.
Les particules enrobées de paracétamol et de tramadol ont les mêmes caractéristiques granulométriques que celles de l'exemple 2.

### 2/ Compression

Les poinçons sont ronds, convexes (rayon de 25 mm) avec un diamètre de 16 mm.

La machine à comprimer (FETTE PT 3090) est équipée de 61 poinçons ronds convexes (rayon de 25 mm) avec un diamètre de 16 mm.

On utilise une lubrification externe de stéarate de magnésium pour lubrifier les poinçons et les matrices.

La couche A (masse de 800 mg) est tassée sous une force de précompression de 2,3 kN.

Le mélange de poudres de la couche B (masse de 200 mg) est ensuite introduit à la surface de la couche A prétassée.

Une force de précompression de 13,0 kN est appliquée avant la compression finale des deux couches successivement fumées, sous une force de 37,1 kN, pour cibler une dureté de 50 N.

93777 comprimés sont préparés avec une vitesse maximale de fabrication des comprimés de 110 000 comprimés/heure.

Les comprimés bicouches ainsi préparés ont une masse théorique de 1000 mg et contiennent une dose de 325 mg de paracétamol et une dose de 37,5 mg de tramadol HCl.

Chaque comprimé a la composition finale suivante (tableau 17) :

**Tableau 17**

| Formule unitaire (mg) | |
|---|---|
| COUCHE A | |
| PARACETAMOL ENROBÉ | 368,5 |
| MANNITOL M300 | 164,5 |
| MANNITOL 60 | 164,5 |
| KOLLIDON CL | 75,2 |
| ASPARTAME | 15,0 |
| ROOTBEER MINT FLAVOUR | 7,5 |
| STEARATE DE MAGNESIUM | 4,8 |
| S/TOTAL COUCHE A | 800,0 |

| COUCHE B | |
|---|---|
| TRAMADOL HCl ENROBÉ | 56,6 |
| MANNITOL M300 | 54,6 |
| MANNITOL 60 | 54,6 |
| KOLLIDON CL | 24,7 |
| ASPARTAME | 5,0 |
| ROOTBEER MINT FLAVOUR | 2,5 |
| GREEN COLOUR | 1,0 |
| STEARATE DE MAGNESIUM | 1,0 |
| S/TOTAL COUCHE B | 200,0 |
| MASSE TOTALE DU COMPRIMÉ | 1000,0 |

Les comprimés ont les caractéristiques physiques et chimiques suivantes (tableau 18) :

**Tableau 18**

| | MOYENNE (CV) |
|---|---|
| Poids (mg) | 991,4 |
| (n = 20) | (0,6%) |
| Dureté (N) | 51,7 |
| (n = 10) | (5,8%) |
| Friabilité (%) | 0,06 |
| (n = 10) | |
| Désintégration en bouche | 20 s |
| (n = 6) | |

## Revendications

1. Comprimé **caractérisé par le fait qu'**il est constitué d'au moins deux couches superposées et solidaires, deux desdites couches comprenant chacune au moins une substance active et **par le fait que** le comprimé présente une dureté comprise entre 1 kp (9.8 N) et 6 kp (58.8 N), une friabilité de moins de 1% en poids et qu'il est destiné à se désintégrer ou à se solubiliser dans la bouche, sans mastication, au contact de la salive, en moins de 60 secondes, formant ainsi une suspension de particules aisée à avaler.

2. Comprimé selon la revendication 1, **caractérisé par le fait qu'**il comprend 2 ou 3 couches.

3. Comprimé selon la revendication 1 ou la revendication 2, **caractérisé en ce que** chaque couche comprend un mélange d'excipients comprenant:
- au moins un agent soluble choisi dans le groupe comprenant les sucres, les polyols présentant moins de 13 atomes de carbone, et leurs mélanges, et
- soit au moins un agent désintégrant soit au moins un agent désintégrant et au moins un agent gonflant.

4. Comprimé selon l'une quelconque des revendications 1 à 3, **caractérisé par le fait qu'**il comprend trois couches, seules les deux couches externes comprenant au moins une substance active.

5. Comprimé selon l'une quelconque des revendications 1 à 4, **caractérisé en ce que** chaque couche comprend en outre un lubrifiant, un agent perméabilisant, un agent antistatique, un diluant insoluble dans l'eau, un liant, un édulcorant, un arôme, un colorant, des adjuvants, seuls ou en mélanges.

6. Comprimé selon la revendication **5**, **caractérisé en ce que** les adjuvants sont choisis dans le groupe comprenant les accélérateurs de désintégration, les agents ajusteurs de pH, les systèmes générateurs de dioxyde de carbone, les tensioactifs, seuls ou en mélanges.

7. Comprimé selon l'une quelconque des revendications **1 à 6, caractérisé par le fait qu'**au moins l'une des substances actives se présente sous une forme à libération modifiée.

8. Comprimé selon l'une quelconque des revendications **1 à 7, caractérisé par le fait qu'**au moins l'une des substances actives se présente sous une forme cristalline, ou sous la forme de noyaux, comportant un enrobage à des fins de masquage de goût.

9. Procédé de préparation du comprimé selon l'une quelconque des revendications **1 à** 8 comprenant les étapes suivantes :
1. préparation d'au moins deux types de particules de substances actives éventuellement enrobées ;
2. préparation d'au moins deux mélanges secs comprenant chacun des excipients de compression et au moins un type de particules de substance active ;
3. pré-compression d'au moins l'un des mélanges de poudres obtenu ci-dessus avec une contrainte de compression de 0,5 à 5 kN;
4. application sur le mélange ci-dessus d'un autre mélange ;
5. précompression éventuelle avec une contrainte de compression de 0,5 à 5 kN ;
6. compression finale sur les couches pré-formées obtenues précédemment avec une contrainte de compression de 5 à 50 kN,
les étapes 4 et 5 pouvant être répétées au moins une fois selon le nombre de couches du comprimé.

## Patentansprüche

1. Tablette, **dadurch gekennzeichnet, dass** sie aus mindestens zwei übereinanderliegenden und miteinander verbundenen Schichten aufgebaut ist, wobei zwei der Schichten jede mindestens einen Wirkstoff umfassen, und dadurch, dass die Tablette eine Härte, die zwischen 1 kp (9.8 N) und 6 kp (58.8 N) liegt, eine Friabilität von weniger als 1 Gew.-% aufweist und dadurch, dass sie dazu bestimmt ist, ohne Kauen beim Kontakt mit dem Speichel in weniger als 60 Sekunden im Mund zu zerfallen oder sich aufzulösen und so eine Partikelsuspension bildet, die leicht zu schlucken ist.

2. Tablette nach Anspruch 1, **dadurch gekennzeichnet**, das sie 2 oder 3 Schichten umfasst.

3. Tablette nach Anspruch 1 oder Anspruch 2, **dadurch gekennzeichnet**, das jede Schicht ein Gemisch von Exzipienten enthält, das umfasst:
- mindestens ein lösliches Agens ausgewählt aus der Gruppe bestehend aus Zuckern, Polyolen, die weniger als 13 Kohlenstoffatome aufweisen, und Gemischen daraus, und
- entweder mindestens ein Sprengmittel oder mindestens ein Sprengmittel und mindestens ein Quellmittel.

4. Tablette nach einem der Ansprüche 1 bis 3, **dadurch gekennzeichnet, dass** sie drei Schichten umfasst, wobei nur die beiden äußeren Schichten mindestens einen Wirkstoff enthalten.

5. Tablette nach einem der Ansprüche 1 bis 4, **dadurch gekennzeichnet**, das jede Schicht des Weiteren ein Gleitmittel, ein Permeabilisierungsmittel, ein Antistatikum, ein in Wasser unlösliches Verdünnungsmittel, ein Bindemittel, einen Süßstoff, einen Geschmacksstoff, einen Farbstoff, Adjuvantien, allein oder im Gemisch enthält.

6. Tablette nach Anspruch 5, **dadurch gekennzeichnet, dass** die Adjuvantien ausgewählt sind aus der Gruppe bestehend aus Zerfallsbeschleunigern, pH-Regulierungsmitteln, Kohlendioxid erzeugenden Systemen, Tensiden, allein oder im Gemisch.

7. Tablette nach einem der Ansprüche 1 bis 6, **dadurch gekennzeichnet, dass** mindestens einer der Wirkstoffe in Form modifizierter Freisetzung vorliegt.

8. Tablette nach einem der Ansprüche 1 bis 7, **dadurch gekennzeichnet, dass** mindestens einer der Wirkstoffe in kristalliner Form vorliegt oder in Form eines Kerns, umfassend einen Überzug zur Geschmacksmaskierung.

9. Verfahren zur Herstellung der Tablette nach einem der Ansprüche 1 bis 8, umfassend die folgenden Schritte:
1. Herstellung mindestens zweier Arten von gegebenenfalls überzogenen Wirkstoffpartikeln;
2. Herstellung mindestens zweier Trockengemische, welche jeweils Exzipienten zur Verdichtung und mindestens eine Art von Wirkstoffpartikeln umfassen;
3. Vorverdichtung mindestens eines der erhaltenen Pulvergemische mit einem Kompressionsdruck von 0,5 bis 5 kN;
4. Aufbringen eines anderen Gemischs auf dem obigen Gemisch;
5. gegebenenfalls Vorverdichtung mit einem Kompressionsdruck von 0,5 bis 5 kN;
6. Endverpressung der zuvor erhaltenen vorgeformten Schichten mit einem Kompressionsdruck von 0,5 bis 50 kN,
wobei die Schritte 4 und 5 abhängig von der Anzahl der Schichten mindestens ein Mal wiederholt werden können.

## Claims

1. Tablet **characterized in that** it consists of at least two superimposed and integral layers, two of the said layers each comprising at least one active substance and wherein said tablet has a hardness of 1 kp (9.8 N) to 6 kp (58.8 N), a friability of less than 1% by weight, and is intended to be disintegrated or dissolved in the mouth, without chewing, on contact with saliva, in less than 60 seconds, forming a particle suspension that is easy to swallow.

2. Tablet according to claim 1, **characterized in that** it comprises 2 or 3 layers.

3. Tablet according to claim 1 or claim 2, **characterized in that** each layer comprises a mixture of excipients comprising:
- at least one soluble agent selected from the group comprising sugars, polyols with less than 13 carbon atoms, and mixtures thereof, and
- either at least one disintegrant, or at least one disintegrating agent and at least one swelling agent.

4. Tablet according to anyone of claims 1 to 3, **characterized in that** it comprises three layers, only the two outer layers comprising at least one active substance.

5. Tablet according to anyone of claims 1 to 4, **characterized in that** each layer further comprises a lubricant, a permeabilizing agent, an antistatic agent, a water-insoluble diluent, a binder, a sweetener, a flavouring, a colorant, adjuvants, alone or as mixtures.

6. Tablet according to claim 5, **characterized in that** the adjuvants are selected from the group comprising disintegration accelerators, pH adjusters, systems for generating carbon dioxide, surfactants, alone or as mixtures.

7. Tablet according to anyone of claims 1 to 6, **characterized in that** at least one of the active substances is in a modified-release form.

8. Tablet according to anyone of claims 1 to 7, **characterized in that** at least one of the active substances is in a crystalline form, or in the form of cores, comprising a coating for the purpose of taste masking.

9. Process for preparing the tablet according to anyone of claims 1 to 8, comprising the following steps:
1. preparation of at least two types of particles of optionally coated active substances ;
2. preparation of at least two dry mixtures each comprising tableting excipients and at least one type of particles of active substance ;
3. pre-compression of at least one of the powder mixtures obtained above with a compression force ranging from 0.5 to 5 kN ;
4. application of another mixture to the above mixture ;
5. optional pre-compression with a compression force ranging from 0.5 to 5 kN ;
6. final compression on the pre-formed layers previously obtained with a compression force ranging from 5 to 50 kN,
steps 4 and 5 possibly being repeated at least once depending on the number of layers of the tablet.
